# EUROPEAN PATENT APPLICATION

(11) **EP 3 088 996 A1**
(43) Date of publication of application: **02.11.2016**
(21) Application number: 16167546.7
(22) Date of filing: 28.04.2016
(51) Int. Cl.: G06F 3/01, A61H 3/06

(54) **SYSTEMS AND METHODS FOR TACTILE GUIDANCE**

(30) Priority: 28.04.2015 US 201514697680
(71) Applicant: Immersion Corporation, San Jose, CA 95134 (US)
(72) Inventor: HAMAM, Abdelwahab, Montreal, Québec H2X 3R5 (CA); CRUZ-HERNANDEZ, Juan Manuel, Montreal, Québec H3Z 1T1 (CA); WU, Liwen, Verdun, Québec H4H 1V6 (CA); SABOUNE, Jamal, Montreal, Québec H2W 2R2 (CA); LEVESQUE, Vincent, Montreal, Québec H2J 2R1 (CA); LACROIX, Robert, San Jose, California 95134 (US); GRANT, Danny, Laval, Québec H7M 2A1 (CA)
(74) Representative: McDougall, Robert Campbell

(57) **Abstract**

Systems (100) and methods for tactile guidance are disclosed. One illustrative method disclosure herein includes: receiving a sensor signal from a sensor (115) configured to determine one or more objects associated with an area; determining area information based in part on the sensor signal; determining a haptic effect based in part on the area information; and transmitting a haptic signal associated with the haptic effect to a haptic output device (118) configured to output the haptic effect.

## Description

### Field of the Invention

The present invention relates to the field of user interface devices. More specifically, the present invention relates to systems for tactile guidance.

### Background

Handheld devices, such as mobile telephones have become increasingly popular. Some of these devices include area sensors. These area sensors enable mobile devices to detect information about surrounding areas. This information may then be communicated to the user via visual means. Many devices further include capability for haptic feedback, which can be used to communicate information to the user via tactile means. Accordingly, there is a need for systems and methods for tactile guidance.

### Summary

Embodiments of the present invention include devices featuring capability to determine haptic signals and output haptic effects. In some embodiments, these haptic effects may comprise surface-based haptic effects that simulate one or more features in a touch area. Features may include, but are not limited to, changes in texture and/or simulation of boundaries, obstacles, or other discontinuities in the touch surface that can be perceived through use of an object in contact with the surface. In some embodiments haptic effects may comprise surface deformations, vibrations, and other tactile effects known in the art. In some embodiments these haptic effects may be used to communicate information associated with an area, for example, information associated with obstacles in the area or a map of the area.

In one embodiment, a method for tactile guidance comprises: receiving a sensor signal from a sensor configured to determine one or more objects associated with an area; determining area information based in part on the sensor signal; determining a haptic effect based in part on the area information; and transmitting a haptic signal associated with the haptic effect to a haptic output device configured to output the haptic effect.
The method may be modified in any suitable way as disclosed herein including, but not limited to, any one or more of the following. The method may further comprise: determining a map based in part on the sensor signal; and, transmitting data associated with the map to a remote database. The method may be configured such that determining the map comprises receiving data about the map from a database. The method may further comprise: simplifying the map to comprise linear distances within the area; identifying one or more obstacles in the area. The method may be configured such that the haptic effect is associated with the one or more obstacles. The method may be configured such that the sensor comprises one or more of an ultrasonic sensor, an infrared sensor, a laser sensor, or a camera. The method may be configured such that the haptic output device comprises one or more of a piezoelectric actuator, an electric motor, an electro-magnetic actuator, a voice coil, a shape memory alloy, an electro-active polymer, a solenoid, an eccentric rotating mass motor (ERM), or a linear resonant actuator (LRA). The method may be configured such that the sensor and haptic output device are both associated with one of a wearable or a graspable device. The method may be configured such that the graspable device comprises one of a mobile device or a cane. The method may be configured such that the wearable device comprises one of a helmet, gloves, glasses, or augmented reality glasses.

In another illustrative embodiment a system for tactile guidance comprises: a sensor configured to determine area information and transmit a sensor signal associated with the area information; a processor in communication with the sensor and configured to: determine area information based in part on the sensor signal; determine a haptic effect based in part on the area information; and transmit a haptic signal associated with the haptic effect; a haptic output device in communication with the processor, the haptic output device configured to receive the haptic signal and output the haptic effect.
The system may be modified in any suitable way as disclosed herein including, but not limited to, any one or more of the following. The system may be configured such that all of the components of the system are associated with a cane.

Another illustrative embodiment comprises a non-transitory computer readable medium comprising program code, which when executed by the processor is configured to cause the processor to: receive a sensor signal from a sensor configured to determine one or more objects associated with an area; determine area information based in part on the sensor signal; determine a haptic effect based in part on the area information; and transmit a haptic signal associated with the haptic effect to a haptic output device configured to output the haptic effect. The non-transitory computer readable medium may be modified in any suitable way as disclosed herein including, but not limited to, any one or more of the following. The non-transitory computer readable medium may further comprise program code, which when executed by the processor is configured to cause the processor to: determine a map based in part on the sensor signal; and, transmit data associated with the map to a remote database. The non-transitory computer readable medium may be configured such that determining the map comprises receiving data about the map from a database. The non-transitory computer readable medium may further comprise program code, which when executed by the processor is configured to cause the processor to: simplify the map to comprise linear distances within the area; identify one or more obstacles in the area. The non-transitory computer readable medium may be configured such that the haptic effect is associated with the one or more obstacles. The non-transitory computer readable medium may be configured such that the sensor comprises one or more of an ultrasonic sensor, an infrared sensor, a laser sensor, or a camera. The non-transitory computer readable medium may be configured such that the haptic output device comprises one or more of a piezoelectric actuator, an electric motor, an electro-magnetic actuator, a voice coil, a shape memory alloy, an electro-active polymer, a solenoid, an eccentric rotating mass motor (ERM), or a linear resonant actuator (LRA). The non-transitory computer readable medium may be configured such that the non-transitory computer readable medium, sensor, and haptic output device are both associated with one of a wearable or a graspable device. The non-transitory computer readable medium may be configured such that the graspable device comprises one of a mobile device or a cane. The non-transitory computer readable medium may be configured such that the wearable device comprises one of a helmet, gloves, glasses, or augmented reality glasses.

These illustrative embodiments are mentioned not to limit or define the limits of the present subject matter, but to provide examples to aid understanding thereof. Illustrative embodiments are discussed in the Detailed Description, and further description is provided there. Advantages offered by various embodiments may be further understood by examining this specification and/or by practicing one or more embodiments of the claimed subject matter.

### Brief Description of the Drawings

A full and enabling disclosure is set forth more particularly in the remainder of the specification. The specification makes reference to the following appended figures.
Figure 1A shows an illustrative system for tactile guidance.
Figure 1B shows an external view of one embodiment of the system shown in Figure 1A.
Figure 1C illustrates an external view of another embodiment of the system shown in Figure 1A.
Figure 2A illustrates an example embodiment for tactile guidance.
Figure 2B illustrates another example embodiment for tactile guidance.
Figure 3A illustrates yet another example embodiment for tactile guidance.
Figure 3B illustrates yet another example embodiment for tactile guidance.
Figure 4 illustrates yet another example embodiment for tactile guidance.
Figure 5 is a flow chart of method steps for one example embodiment for tactile guidance.

### Detailed Description

Reference will now be made in detail to various and alternative illustrative embodiments and to the accompanying drawings. Each example is provided by way of explanation, and not as a limitation. It will be apparent to those skilled in the art that modifications and variations can be made. For instance, features illustrated or described as part of one embodiment may be used in another embodiment to yield a still further embodiment. Thus, it is intended that this disclosure include modifications and variations as come within the scope of the appended claims and their equivalents.

### Illustrative Example of a Device for Tactile Guidance

One illustrative embodiment of the present disclosure comprises a mobile electronic device, such as a tablet, e-reader, mobile phone, wearable device, or computer such as a laptop computer. In the illustrative embodiment, the electronic device comprises a display (such as a touch-screen display), a memory, and a processor in communication with each of these elements. The illustrative device comprises applications commonly found on mobile devices, e.g., applications for texting, email, games, etc. A user of the illustrative device may use these applications while engaging in other activities that require the user's focus, e.g., while walking through a room or on a crowded street.

In the illustrative embodiment, the mobile device further comprises an area sensor configured to detect one or more objects in the area surrounding the device and transmit a sensor signal associated with these objects. In the illustrative embodiment, the one or more objects may comprise any object found in an area around the user, e.g., indoor objects (e.g., furniture, supporting posts, walls, doors, or other objects associated with an indoor area); outdoor objects (e.g., trees, rocks, holes, roots, stumps, curbs, cars, bicycles, or other objects associated with an outdoor area). Further, the one or more objects may comprise moving objects (e.g., animals, cars, people, etc.) or non-moving objects (e.g., trees, walls, puddles, etc.) In the illustrative embodiment the area sensor may comprise one or more of an ultrasonic sensor, an infrared sensor, a laser sensor, or a camera. In some embodiments, the area sensor may further be configured to detect one or more key descriptors of the one or more objects. In some embodiments these key descriptors may comprise, e.g., color, temperature, movement, acceleration, dimensions, or some other characteristic associated with the object.

In the illustrative embodiment, the processor may receive signals from the area sensor. Based on these signals the processor may determine one or more objects associated with the area around the user and one or more key descriptors associated with these objects. For example, in the illustrative embodiment, the area sensor may detect various objects of the area around the user, e.g., an indoor or outdoor area in which the user is located. In the illustrative embodiment the mobile device may determine the user's location based in part on the objects in the area. For example, the mobile device may compare data associated with objects to a database of area information. Based on this comparison the mobile device may determine that the mobile device and user are in a known location, e.g., home, a mall, a park, a campus, an office, or some other known location. In such an embodiment, the database may comprise an Internet accessible "cloud" database, which may be continuously updated by one or more public and/or private groups. Further, such a database may comprise a database of map data used or accessed by a plurality of mobile applications and webpages. In some embodiments, this database may comprise area information associated with various areas throughout the planet. In the illustrative embodiment, the processor may compare this area information with information detected around the user and, based in part on this comparison, determine the user's present location.

In the illustrative embodiment, the user may be focused on a display of the mobile device while traversing an area (e.g., a room, a hallway, or a sidewalk). For example, the user may be focused on a texting application and therefore not focused on his or her surroundings. In such an embodiment the mobile device may determine that the user is likely to walk into one or more objects, e.g., the object(s) may comprise an obstacle in the user's path, e.g., a chair. In such an embodiment the mobile device may output an alert to warn the user. In the illustrative embodiment this alert may comprise one or more of a graphical alert (e.g., a visible warning on the display), an audible alert (e.g., an audible alarm), or a tactile alert (e.g., a vibration, deformation, surface feature, or some other haptic effect). For example, in the illustrative embodiment, if the mobile device determines that the user is within a certain distance of an obstacle, the mobile device may output two alerts. In the illustrative embodiment, these two alerts may comprise a graphical alert, such as displaying text on the display of the mobile device, and a tactile alert. In some embodiments the tactile alert may comprise, e.g., a vibration, a deformation (e.g., a deformation of a surface in the direction of the object or in a direction away from the object), or a surface based effect (e.g., a change in the coefficient of friction on a touch surface). In the illustrative embodiment, these alerts may notify the user that he or she is about to walk into the obstacle.

Further, in the illustrative embodiment, the processor may determine a "map" of the area in which the user is located. This map may comprise a layout of the area and the various objects located in the area. In the illustrative embodiment the processor may store this map in a local memory. Further, in such an embodiment, the processor may determine haptic effects associated with the one or more objects in the area. In such an embodiment, the illustrative device may determine one or more haptic effects configured to alert the user to the location of the objects. In some embodiments these haptic effects may be output as surface based effects, for example, on a display of the mobile device. These effects may allow the user to locate objects, such as obstacles, within a room by interacting with the surface of the display. For example, surface objects such as variations in texture or coefficient of friction may indicate the location of various objects. Alternatively, the processor may output the haptic effect as the user approaches an object, thus alerting the user that the object is an obstacle in the user's path.

In the illustrative embodiment, the mobile device may be configured to store a simplified map. For example, the mobile device may determine a distance and angle from the user's present location to one or more objects in the area. In the illustrative embodiment, the simplified map may be used to identify objects that the user is approaching, e.g., as the user moves from one position to another the mobile device may detect, based on the simplified map, that the user is approaching an object (e.g., a hallway or a door).

Further, in the illustrative embodiment, the mobile device may be configured to direct the user to a location. For example, in the illustrative embodiment, the mobile device may comprise mapping or location assistance applications configured to direct the user from one location to another. In the illustrative embodiment, these applications may be configured to utilize Satellite Positioning Systems, e.g., GPS, AGPS, GLONASS, Galileo, etc., to determine the user's present location. In other embodiments, the mobile device may rely on other information to determine the user's present location. For example, the mobile device may determine the user's location based on objects detected using an area sensor. Further, the mobile device may determine that the user should change direction based on these objects.

In the illustrative embodiment, the mobile device may determine haptic effects to guide the user along a route (e.g., an indoor route or an outdoor route). For example, in the illustrative embodiment, the mobile device may output haptic effects to guide the user to a location. For example, in such an embodiment, a haptic effect on the right side of the mobile device may indicate that the user should make a right turn and a haptic effect on the left side of the mobile device may indicate that the user should make a left turn. In some embodiments these haptic effects may comprise, e.g., a vibration, a surface deformation (e.g., a deformation in the direction toward which the user should turn), or a surface based effect (e.g., a change in coefficient of friction or perceived surface texture). Further, in such an embodiment, the mobile device may output one or more alerts to alert the user that the user is about to encounter an obstacle. In some embodiments, the alert may be used by a user with a visual impairment or a user who is distracted, e.g., a user who is sending a text or typing an email while walking.

In another illustrative embodiment, the illustrative device may comprise a device for use by a visually impaired person. In one embodiment, this device may comprise a haptic cane. In such an embodiment, the haptic cane may comprise an area sensor (e.g., an ultrasonic sensor, an infrared sensor, a laser sensor, or a camera) and an array of haptic output devices. In the illustrative embodiment, the haptic cane may be configured to perform the operations discussed above. For example, the haptic cane may be configured to detect obstacles in the user's path and output haptic effects to alert the user of the obstacles. In the illustrative embodiment, these haptic effects may comprise effects configured to alert the user to the type of obstacle, e.g., a wall may comprise one haptic effect and a door may comprise a different haptic effect. In some embodiments this alert may be based in part on one or more key descriptors associated with the one or more objects. Further, in the illustrative embodiment, the haptic cane may comprise functionality to alert the user of a route or pathway from one location to another. For example, the haptic cane may be configured to output haptic effects to alert the user to move forward, backward, left, or right.

In some embodiments the illustrative device may be configured to determine whether to output an alert based on one or more key descriptors associated with a detected object. In such an embodiment, the key descriptors may comprise, e.g., the object's size, direction of movement, speed of movement, distance from the user, relative danger, or some other key descriptor associated with the object. For example, if the detected object is relatively distant (e.g., more than a threshold distance), the illustrative device may output no alert. Similarly, the illustrative device may determine additional information, about the object, e.g., the object's size, its relative danger, its speed of movement, its direction of movement, and based on this information determine whether to output an alert to the user.

Further, in some embodiments one or more objects in the area around the user may comprise active objects. For example, active objects may comprise objects with processing and data transfer capabilities. These active objects may be configured to communicate with the illustrative device or a server accessible via a network such as the Internet and provide information about the area around the active object. In some embodiments this information may comprise information about the active object, other objects in the area, and/or the user's movement to the illustrative device. The illustrative device may use these signals to determine whether to output an alert to the user and/or what type of alert to output to the user. For example, in the illustrative embodiment, active objects may comprise dedicated sensors, mobile devices comprising area sensors, or other electronic devices comprising area sensors. In the illustrative embodiment, these active objects may periodically or substantially continuously detect area information and transmit signals associated with this area information. These signals may be uploaded to a database accessible to other mobile devices, e.g., a cloud database accessible via the Internet. Alternatively, in some embodiments, active objects may be configured to transmit data directly to other devices, e.g., other mobile devices in the same area as the active object.

These illustrative embodiments are mentioned not to limit or define the limits of the present subject matter, but to provide examples to aid understanding thereof. Illustrative embodiments are discussed in the Detailed Description, and further description is provided there. Advantages offered by various embodiments may be further understood by examining this specification and/or by practicing one or more embodiments of the claimed subject matter.

### Illustrative Systems for Tactile guidance

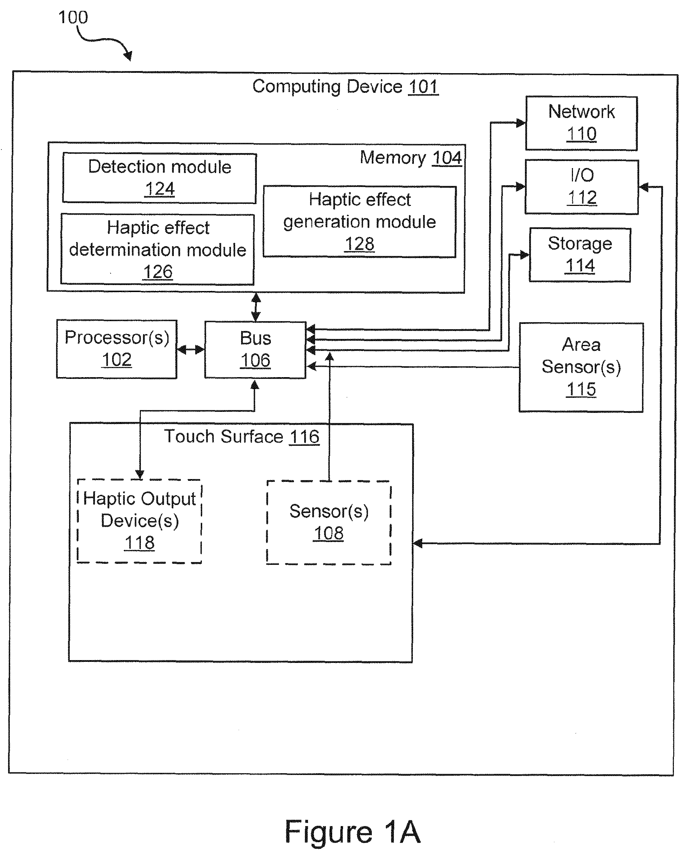

Figure 1A shows an illustrative system 100 for tactile guidance. Particularly, in this example, system 100 comprises a computing device 101 having a processor 102 interfaced with other hardware via bus 106. A memory 104, which can comprise any suitable tangible (and non-transitory) computer-readable medium such as RAM, ROM, EEPROM, or the like, embodies program components that configure operation of the computing device. In this example, computing device 101 further includes one or more network interface devices 110, input/output (I/O) interface components 112, and additional storage 114.

Network device 110 can represent one or more of any components that facilitate a network connection. Examples include, but are not limited to, wired interfaces such as Ethernet, USB, IEEE 1394, and/or wireless interfaces such as IEEE 802.11, Bluetooth, or radio interfaces for accessing cellular telephone networks (e.g., transceiver/antenna for accessing a CDMA, GSM, UMTS, or other mobile communications network).

I/O components 112 may be used to facilitate connection to devices such as one or more displays, keyboards, mice, speakers, microphones, cameras (e.g., a front and/or a rear facing camera on a mobile device), Satellite Positioning System Receivers (e.g., GPS, AGPS, GLONASS, Galileo, etc.) and/or other hardware used to input data or output data. Storage 114 represents nonvolatile storage such as magnetic, optical, or other storage media included in device 101.

Area sensor(s) 115 comprise one or more devices configured to detect objects associated with the area around the user and transmit signals associated with these objects to the processor(s) 102. For example, area sensor(s) 115 may comprise one or more of an ultrasonic sensor, an infrared sensor, a laser sensor, or a camera (e.g., an optical or an infrared camera). In one embodiment, area sensor(s) 115 may comprise an MB1320 XL-MaxSonar-AE2, however, in other embodiments many other types and models of area sensor(s) may be used as well or alternatively. In some embodiments, the objects detected by area sensor(s) 115 may comprise any object found in an area around the user, e.g., indoor objects (e.g., furniture, supporting posts, walls, doors, or other objects associated with an indoor area); outdoor objects (e.g., trees, rocks, holes, roots, stumps, curbs, cars, bicycles, or other objects associated with an outdoor area). Further, the objects may comprise moving objects (e.g., animals, cars, people, etc.) or non-moving objects (e.g., trees, walls, puddles, etc.). In some embodiments, the area sensor may further be configured to detect one or more key descriptors of the one or more objects. In some embodiments these key descriptors may comprise, e.g., color, temperature, movement, acceleration, dimensions, or some other characteristic associated with the object. For example, in some embodiments, the area sensor 115 may comprise an infrared camera. In some embodiments, the infrared camera may detect thermal characteristics of one or more objects. In some embodiments, thermal characteristics may be used to detect whether an object is living (e.g., a person or animal) or the threat level of an object, e.g., a fire or a hot plate.

In some embodiments, one or more objects in the area around the user may comprise active objects. For example, active objects may comprise objects with processing and data transfer capabilities. These active objects may be configured to communicate with the system 100 and provide information about the active object, other objects in the area, and or the user's movement to the device 100. In some embodiments these signals may be received by area sensor(s) 115. Further, in some embodiments the processor 102 of the device 100 may use these signals to determine whether to output an alert to the user and/or what type of alert to output to the user. For example, in the illustrative embodiment, active objects may comprise dedicated sensors, mobile devices comprising area sensors, or other electronic devices comprising area sensors. In the illustrative embodiment, these active objects may periodically or substantially continuously detect area information and transmit signals associated with this area information. These signals may be uploaded to a database accessible to other mobile devices, e.g., a cloud database accessible via the Internet. Thus, in some embodiments, the data detected by active objects may be stored and accessible by computing devices for some time after the data is detected (e.g., minutes, hours, days, weeks, or years, etc.). Alternatively, in some embodiments, active objects may be configured to transmit data directly to other devices, e.g., other mobile devices in the same area as the active object.

In one embodiment the system 100 discovers the active objects in an area around the device 100 based on signal data, e.g., GPS, wi-fi, or cellular signals. In some embodiments, this data may comprise data associated with the location of the active objects and data associated with the location of the device 100. In another embodiment, a central system/server receives data associated with device 100 and the active objects and determines based on this data the active objects that are in the area surrounding the device 100. In some embodiments, the device 100 and the active objects may communicate directly wirelessly (e.g. via wi-fi, Bluetooth, etc.). In other embodiments, the device 100 and the active objects may communicate through a central system/server accessible via a network. Further, in some embodiments, the active objects may comprise one or more other users' devices in the area (e.g., other devices similar to device 100).

System 100 further includes a touch surface 116, which, in this example, is integrated into device 101. Touch surface 116 represents any surface that is configured to sense touch input of a user. One or more sensors 108 may be configured to detect a touch in a touch area when an object contacts a touch surface and provide appropriate data for use by processor 102. Any suitable number, type, or arrangement of sensors can be used. For example, resistive and/or capacitive sensors may be embedded in touch surface 116 and used to determine the location of a touch and other information, such as pressure. As another example, optical sensors with a view of the touch surface may be used to determine the touch position. In some embodiments, sensor 108 and touch surface 116 may comprise a touch-screen or a touch-pad. For example, in some embodiments, touch surface 116 and sensor 108 may comprise a touch-screen mounted overtop of a display configured to receive a display signal and output an image to the user. In other embodiments, the sensor 108 may comprise an LED detector. For example, in one embodiment, touch surface 116 may comprise an LED finger detector mounted on the side of a display. In some embodiments, the processor is in communication with a single sensor 108, in other embodiments, the processor is in communication with a plurality of sensors 108, for example, a first touch screen and a second touch screen.

In some embodiments, one or more sensor(s) 108 further comprise one or more sensors configured to detect movement of the mobile device (e.g., accelerometers, gyroscopes, cameras, GPS, or other sensors). These sensors may be configured to detect user interaction that moves the device in the X, Y, or Z plane, for example, when the user carries the mobile device through an area. The sensor 108 is configured to detect user interaction, and based on the user interaction, transmit signals to processor 102.

In some embodiments, sensor 108 may be configured to detect multiple aspects of the user interaction. For example, sensor 108 may detect the speed and pressure of a user interaction, and incorporate this information into the interface signal. Further, in some embodiments, the user interaction comprises a multi-dimensional user interaction away from the device. For example, in some embodiments a camera associated with the device may be configured to detect user movements, e.g., hand, finger, body, head, eye, or feet motions or interactions with another person or object.

In the example shown in Figure 1A, a haptic output device 118 in communication with processor 102 is coupled to touch surface 116. In some embodiments, haptic output device 118 is configured to output a haptic effect simulating a texture on the touch surface in response to a haptic signal. Additionally or alternatively, haptic output device 118 may provide vibrotactile haptic effects that move the touch surface in a controlled manner. Some haptic effects may utilize an actuator coupled to a housing of the device, and some haptic effects may use multiple actuators in sequence and/or in concert. For example, in some embodiments, a surface texture may be simulated by vibrating the surface at different frequencies. In such an embodiment haptic output device 118 may comprise one or more of, for example, a piezoelectric actuator, an electric motor, an electro-magnetic actuator, a voice coil, a shape memory alloy, an electro-active polymer, a solenoid, an eccentric rotating mass motor (ERM), or a linear resonant actuator (LRA). In some embodiments, haptic output device 118 may comprise a plurality of actuators, for example an ERM and an LRA.

In some embodiments, one or more haptic output devices may be configured to output forces in the X, Y, or Z plane with respect to the device. In some embodiments, these effects may be configured to simulate the feeling of an object within the display moving. For example, in one embodiment, a multidimensional haptic effect may be configured to simulate an object (such as a moving object) moving in the X-plane (left or right), the Y-plane (up or down), the Z-plane (into or out of the display), or vectors in these planes. These multi-dimensional haptic effects may simulate features in the touch surface.

Although a single haptic output device 118 is shown here, embodiments may use multiple haptic output devices of the same or different type to output haptic effects, e.g., to simulate surface textures on the touch surface. For example, in one embodiment, a piezoelectric actuator may be used to displace some or all of touch surface 116 vertically and/or horizontally at ultrasonic frequencies, such as by using an actuator moving at frequencies greater than 20 - 25 kHz in some embodiments. In some embodiments, multiple actuators such as eccentric rotating mass motors and linear resonant actuators can be used alone or in concert to provide different textures and other haptic effects.

In still other embodiments, haptic output device 118 may use electrostatic attraction, for example by use of an electrostatic surface actuator, to simulate a texture on the surface of touch surface 116. Similarly, in some embodiments haptic output device 118 may use electrostatic attraction to vary the friction the user feels on the surface of touch surface 116. For example, in one embodiment, haptic output device 118 may comprise an electrostatic display or any other device that applies voltages and currents instead of mechanical motion to generate a haptic effect. In such an embodiment, an electrostatic actuator may comprise a conducting layer and an insulating layer. In such an embodiment, the conducting layer may be any semiconductor or other conductive material, such as copper, aluminum, gold, or silver. And the insulating layer may be glass, plastic, polymer, or any other insulating material.

The processor 102 may operate the electrostatic actuator by applying an electric signal to the conducting layer. The electric signal may be an AC signal that, in some embodiments, capacitively couples the conducting layer with an object near or touching touch surface 116. In some embodiments, the AC signal may be generated by a high-voltage amplifier. In other embodiments the capacitive coupling may simulate a friction coefficient or texture on the surface of the touch surface 116. For example, in one embodiment, the surface of touch surface 116 may be smooth, but the capacitive coupling may produce an attractive force between an object near the surface of touch surface 116. In some embodiments, varying the levels of attraction between the object and the conducting layer can vary the simulated texture on an object moving across the surface of touch surface 116 or vary the coefficient of friction felt as the object moves across the surface of touch surface 116. Furthermore, in some embodiments, an electrostatic actuator may be used in conjunction with traditional actuators to vary the simulated texture on the surface of touch surface 116. For example, the actuators may vibrate to simulate a change in the texture of the surface of touch surface 116, while at the same time; an electrostatic actuator may simulate a different texture, or other effects, on the surface of touch surface 116 or on another part of the computing device 101 (e.g., its housing or another input device).

One of ordinary skill in the art will recognize that multiple techniques may be used to output haptic effects such as varying the coefficient of friction or simulating a texture on a surface. For example, in some embodiments, a texture may be simulated or output using a flexible surface layer configured to vary its texture based upon contact from a surface reconfigurable haptic substrate (including, but not limited to, e.g., fibers, nanotubes, electroactive polymers, piezoelectric elements, or shape memory alloys) or a magnetorheological fluid. In another embodiment, surface texture may be varied by raising or lowering one or more surface features, for example, with a deforming mechanism, air or fluid pockets, local deformation of materials, resonant mechanical elements, piezoelectric materials, micro-electromechanical systems ("MEMS") elements, thermal fluid pockets, MEMS pumps, variable porosity membranes, or laminar flow modulation.

In some embodiments, an electrostatic actuator may be used to generate a haptic effect by stimulating parts of the body near or in contact with the touch surface 116. For example, in some embodiments, an electrostatic actuator may stimulate the nerve endings in the skin of a user's finger or components in a stylus that can respond to the electrostatic actuator. The nerve endings in the skin, for example, may be stimulated and sense the electrostatic actuator (e.g., the capacitive coupling) as a vibration or some more specific sensation. For example, in one embodiment, a conducting layer of an electrostatic actuator may receive an AC voltage signal that couples with conductive parts of a user's finger. As the user touches the touch surface 116 and moves his or her finger on the touch surface, the user may sense a texture of prickliness, graininess, bumpiness, roughness, stickiness, or some other texture.

Turning to memory 104, exemplary program components 124, 126, and 128 are depicted to illustrate how a device can be configured in some embodiments to provide tactile guidance. In this example, a detection module 124 configures processor 102 to process signals received from area sensor(s) 115 and determine objects in the area surrounding the user. For example, detection module may configure the processor 102 to receive signals from area sensors (115) as the user moves through an area. Based on these sensor signals the processor 102 may continuously update data associated with the user's location in relation to one or more objects in the area (e.g., walls, hallways, curbs, other people, etc.)

In some embodiments, detection module 124 and processor 102 may determine information associated with a map by comparing detected objects to data in a database, e.g., a locally stored database or a remote database accessed via a network connection. For example, in some embodiments the processor may determine the map by receiving data from a remote database accessible via the Internet. In some embodiments, such a database may comprise a "cloud" database, which may be continuously updated by one or more public or private groups. Further, such a database may comprise a database of map data used or accessed by a plurality of mobile applications and webpages. Further, in some embodiments, this database may comprise area information associated with various areas throughout the planet. In the illustrative embodiment, the processor may compare this area information with information detected around the user to determine the user's present location. Thus, in some embodiments, the processor may determine a map by comparing information about the area around the user (e.g., information about the objects in the area) to information stored in a database. In some embodiments, the processor 102 may determine the user's current location by accessing the database.

Haptic effect determination module 126 represents a program component that analyzes data regarding objects to select a haptic effect to generate. For example, in one embodiment, module 126 comprises code that determines, based on the location of the object, a haptic effect to generate. For example, haptic effect determination module 126 may comprise one or more preloaded haptic effects, which may be selected by the user. These haptic effects may comprise any type of haptic effect that haptic output device(s) 118 are capable of generating. Further, in some embodiments, module 126 may comprise program code configured to manipulate characteristics of a haptic effect, e.g., the effect's intensity, frequency, duration, duty cycle, or any other characteristic associated with a haptic effect. In some embodiments, module 126 may comprise program code to allow the user to manipulate these characteristics, e.g., via a graphical user interface.

Further, in some embodiments, module 126 may comprise program code configured to determine haptic effects based on user interactions. For example, module 126 may be configured to monitor user input on touch surface 116 or other sensors, such as inertial sensors, configured to detect motion of the mobile device. Module 126 may detect this input and generate a haptic effect based on the input. For example, in some embodiments module 126 may be configured to determine a haptic effect configured to simulate the user interaction.

Haptic effect generation module 128 represents programming that causes processor 102 to generate and transmit a haptic signal to haptic output device 118, which causes haptic output device 118 to generate the selected haptic effect. For example, generation module 128 may access stored waveforms or commands to send to haptic output device 118. As another example, haptic effect generation module 128 may receive a desired type of texture and utilize signal processing algorithms to generate an appropriate signal to send to haptic output device 118. As a further example, a desired texture may be indicated along with target coordinates for the haptic effect and an appropriate waveform sent to one or more actuators to generate appropriate displacement of the surface (and/or other device components) to provide the haptic effect. Some embodiments may utilize multiple haptic output devices in concert to output a haptic effect. For instance, a variation in texture may be used to simulate crossing a boundary between a button on an interface while a vibrotactile effect simulates that a button was pressed.

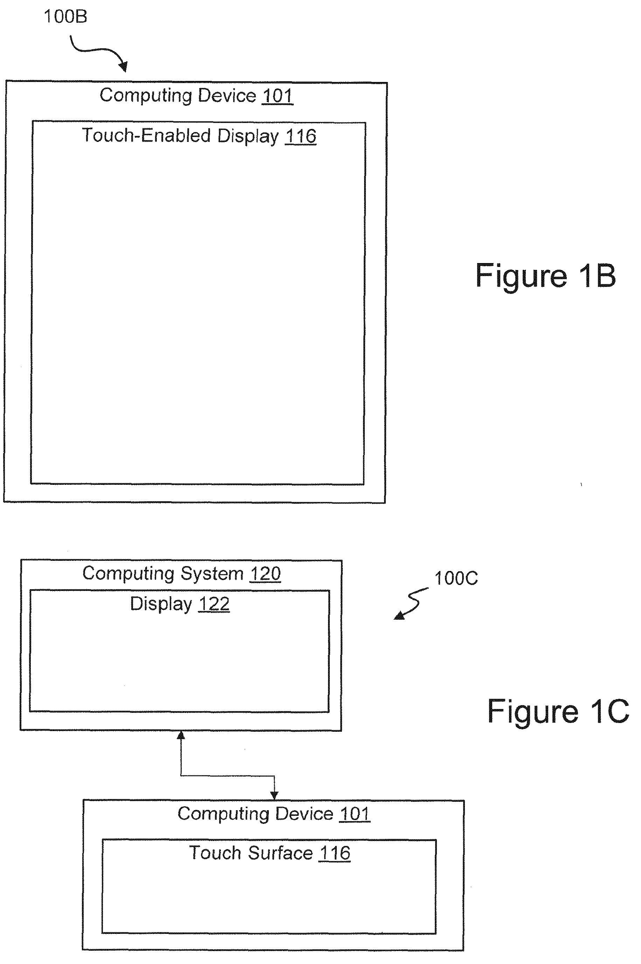

A touch surface may or may not overlay (or otherwise correspond to) a display, depending on the particular configuration of a computing system. In Figure 1B, an external view of a computing system 100B is shown. Computing device 101 includes a touch enabled display 116 that combines a touch surface and a display of the device. The touch surface may correspond to the display exterior or one or more layers of material above the actual display components.

Figure 1C illustrates another example of a touch enabled computing system 100C in which the touch surface does not overlay a display. In this example, a computing device 101 comprises a touch surface 116 which may be mapped to a graphical user interface provided in a display 122 that is included in computing system 120 interfaced to device 101. For example, computing device 101 may comprise a mouse, trackpad, or other device, while computing system 120 may comprise a desktop or laptop computer, set-top box (e.g., DVD player, DVR, cable television box), or another computing system. As another example, touch surface 116 and display 122 may be disposed in the same device, such as a touch enabled trackpad in a laptop computer featuring display 122. Whether integrated with a display or otherwise, the depiction of planar touch surfaces in the examples herein is not meant to be limiting. Other embodiments include curved or irregular touch enabled surfaces that are further configured to provide surface-based haptic effects.

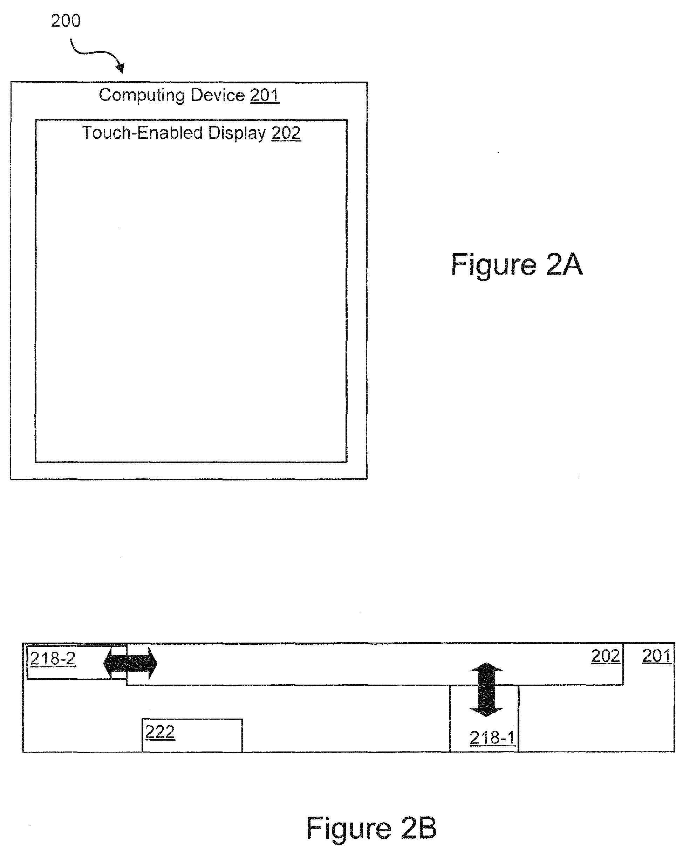

Figures 2A-2B illustrate an example embodiment of a device for tactile guidance. Figure 2A is a diagram illustrating an external view of a system 200 comprising a computing device 201 that comprises a touch enabled display 202. Figure 2B shows a cross-sectional view of device 201. Device 201 may be configured similarly to device 101 of Figure 1A, though components such as the processor, memory, sensors, and the like are not shown in this view for purposes of clarity.

As can be seen in Figure 2B, device 201 comprises a plurality of haptic output devices 218 and an additional haptic output device 222. Haptic output device 218-1 may comprise an actuator configured to impart vertical force to display 202, while 218-2 may move display 202 laterally. In this example, the haptic output devices 218, 222 are coupled directly to the display, but it should be understood that the haptic output devices 218, 222 could be coupled to another touch surface, such as a layer of material on top of display 202. Furthermore, it should be understood that one or more of haptic output devices 218 or 222 may comprise an electrostatic actuator, as discussed above. Furthermore, haptic output device 222 may be coupled to a housing containing the components of device 201. In the examples of Figures 2A-2B, the area of display 202 corresponds to the touch area, though the principles could be applied to a touch surface completely separate from the display.

In one embodiment, haptic output devices 218 each comprise a piezoelectric actuator, while additional haptic output device 222 comprises an eccentric rotating mass motor, a linear resonant actuator, or another piezoelectric actuator. Haptic output device 222 can be configured to provide a vibrotactile haptic effect in response to a haptic signal from the processor. The vibrotactile haptic effect can be utilized in conjunction with surface-based haptic effects and/or for other purposes. For example, each actuator may be used in conjunction to simulate a texture on the surface of display 202.

In some embodiments, either or both haptic output devices 218-1 and 218-2 can comprise an actuator other than a piezoelectric actuator. Any of the actuators can comprise a piezoelectric actuator, an electromagnetic actuator, an electroactive polymer, a shape memory alloy, a flexible composite piezo actuator (e.g., an actuator comprising a flexible material), electrostatic, and/or magnetostrictive actuators, for example. Additionally, haptic output device 222 is shown, although multiple other haptic output devices can be coupled to the housing of device 201 and/or haptic output devices 222 may be coupled elsewhere. Device 201 may comprise multiple haptic output devices 218-1 / 218-2 coupled to the touch surface at different locations, as well.

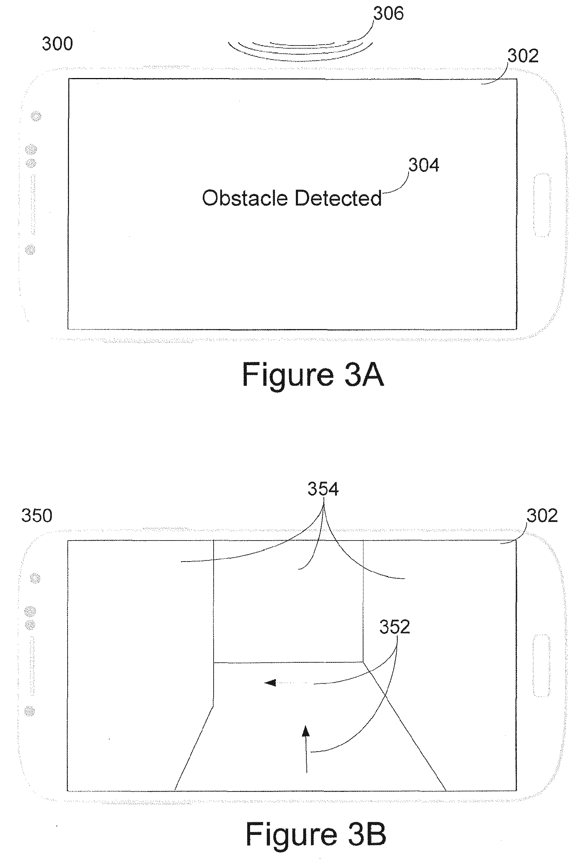

Turning now to Figure 3A, Figure 3A comprises an embodiment for tactile guidance according to one embodiment of the present disclosure. The embodiment shown in Figure 3A comprises a computing device 300. As shown in Figure 3A, computing device 300 comprises a touch-screen display 302. Further, the computing device 300 comprises an area sensor of the type described above, e.g., one or more of an ultrasonic sensor, an infrared sensor, a laser sensor, or a camera. In the embodiment shown in Figure 3A, the computing device may execute a mobile application (e.g., a mobile game, a texting application, an email application, a social media application, etc.). A user may focus on this application while performing other tasks that require the user's attention, e.g., walking or running in an area with obstacles.

In the embodiment shown in Figure 3A, a software program configured to execute a method for tactile guidance may run in the background while the user uses another application. This program may continuously monitor signals received from the area sensor of the mobile device 300. When the area sensor detects an obstacle in the user's path the mobile device 300 outputs an alert to the user. This alert may comprise an audible, visual, or haptic alert. As shown in Figure 3A, this alert comprises a graphical alert and a haptic alert (e.g., a vibration). The graphical alert 304 comprises text that reads "Obstacle Detected." In the embodiment shown in Figure 3A, the graphical alert 304 overlays the display of the mobile device, including the display of whatever mobile application with which the user was interacting. In other embodiments, rather than overlaying the entire display 302, the graphical alert 304 may appear in only the background. Further, as shown in Figure 3A, computing device 300 outputs a haptic effect such as a strong vibration. In other embodiments, the mobile device 300 may be configured to output an audible alert, e.g., an alarm, which may further alert the user that an obstacle is in the user's path.

Turning now to Figure 3B, Figure 3B comprises an embodiment for tactile guidance according to one embodiment of the present disclosure. The embodiment shown in Figure 3B comprises a computing device 350. As shown in Figure 3B, computing device 350 comprises a touch-screen display 302. In the embodiment shown in Figure 3B, the display shows a route 352 along which the user is traveling. In the embodiment shown in Figure 3B, the mobile device 350 comprises an area sensor of the type described above, e.g., one or more of an ultrasonic sensor, an infrared sensor, a laser sensor, or a camera. As the user travels along the route 352 the mobile device outputs haptic effects to alert the user of the route 352 and turns the user should take to follow the route 352. For example, in some embodiments, if the mobile device 350 determines that the user should continue traveling forward the mobile device will output a haptic effect on the front side of the mobile device 350. Similarly, if the mobile device 350 determines that the user should turn left or right the mobile device 350 outputs haptic effects on its right or left side. These haptic effects may alert the user of the direction the user should turn. In some embodiments, this may be helpful to a user who is distracted, e.g., a user who is sending a text or a user who suffers from a visual impairment.

Further, in the embodiment shown in Figure 3B, the mobile device 350 may continuously monitor signals from the area sensor to determine if an obstacle is in the user's path. If an obstacle is detected in the user's path the mobile device 350 outputs an alert, such as a haptic alert, to alert the user that an obstacle is in the user's path.

Further, in the embodiment shown in Figure 3B, based on signals from the area sensor(s) the mobile device 350 may determine a map of the user's surrounding area. This map may comprise the distance and angle between the user and one or more objects in the area. For example, in the embodiment shown in Figure 3B, the mobile device may determine a map that represents the user's angle and distance from each of walls 354. In other embodiments the mobile device 350 may determine a map of data representing the user's angle and distance to additional objects (e.g., trees, people, animals, furniture, etc.). As the user moves along the path 352 and/or as objects in the area move, the mobile device may continuously update this map to maintain accurate data of the user's surroundings.

In the embodiment shown in Figure 3B the mobile device 350 may be configured to output surface based haptic effects such as textures and frictions on the surface of touchscreen display 302. In some embodiments, the mobile device 350 may be configured to output surface based effects associated with the map. For example, in some embodiments, the user may interact with the surface of touchscreen display 302 and the mobile device 350 may output a haptic effect associated with the user's distance between various objects in the user's area. For example, in one embodiment, the haptic effect may comprise a texture or friction of various magnitudes based on the distance the user is from obstacles in each direction. For example, in such an embodiment, if an object is a short distance in front of the user, the mobile device 350 may output a course texture at the front of the touchscreen display 302. In such an embodiment, if an object is a long distance to the user's right the mobile device 350 may output a fine texture on the right side of touchscreen display 302.

In other embodiments the mobile device 350 may output other haptic effects (e.g., vibrations or surface deformations (e.g., the effect may be configured to contract or expand the sides of the device to indicate guidance)). In still other embodiments, one or more of the haptic effects may be output onto the housing of mobile device 350 rather than on touchscreen display 302. Further, in some embodiments the haptic effect may be independent of the objects shown in touchscreen display 302.

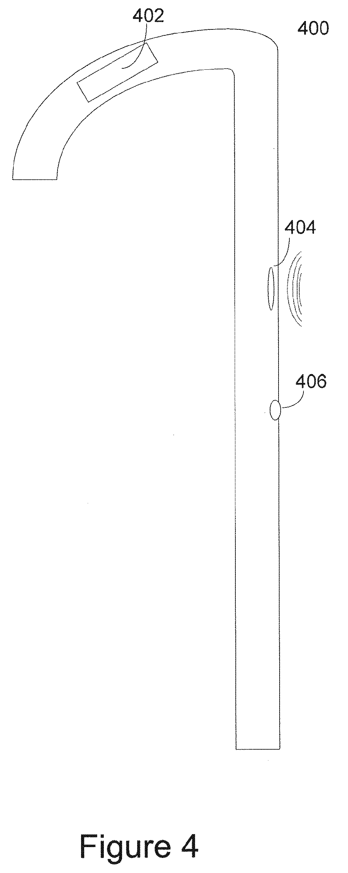

Turning now to Figure 4, Figure 4 comprises an embodiment for tactile guidance according to one embodiment of the present disclosure. In the embodiment shown in Figure 4 the system 400 comprises a haptic cane for use by a visually impaired person. In other embodiments the system may take a different form, e.g., a crutch, a wheelchair, a scooter, a walking aid, or some other form factor. In some embodiments, the device may comprise a device for use by people that must operate in areas with reduced vision, e.g., fireman, police, or soldiers. Thus, in some embodiments the device may comprise a wearable device such as gloves, a jacket, a helmet, glasses, or augmented reality glasses, shoes, socks, a watch, or some other type of wearable device.

In the embodiment shown in Figure 4 the haptic cane 400 comprises a haptic array 402 and two area sensors 404 and 406. The haptic array 402 comprises one or more haptic output devices, e.g., a piezoelectric actuator, an electric motor, an electro-magnetic actuator, a voice coil, a shape memory alloy, an electro-active polymer, a solenoid, an eccentric rotating mass motor (ERM), a linear resonant actuator (LRA), or a haptic output device configured to output surface based effects. The two area sensors 404 and 406 comprise, e.g., an ultrasonic sensor, an infrared sensor, a laser sensor, or a camera. In some embodiments the haptic cane 400 may comprise only one area sensor. In other embodiments the haptic cane 400 may comprise more than two area sensors.

As shown in Figure 4, the haptic cane 400 may be configured to perform the operations discussed herein. For example, based on signals received from area sensors 404 and 406 a processor associated with the haptic cane 400 may be configured to detect obstacles in the users path and output haptic effects to alert the user of the obstacles. In some embodiments, the haptic cane 400 may comprise its own microprocessor. Further, in some embodiments the haptic cane 400 may be configured to communicate with the processor in a mobile device. In such an embodiment, the mobile device may receive sensor signals from the haptic cane and determine haptic effects, which are output by either the mobile device or the haptic cane 400. For example, in some embodiments, the haptic cane 400 may comprise a mount that enables the user to couple a mobile device to the haptic cane. In such an embodiment, rather than area sensors 404 and 406, the processor may instead receive data from area sensors associated with the mobile device. Further, in some embodiments, both the area sensors associated with the mobile device and area sensors associated with the haptic cane 400 may be used to detect objects. Similarly, haptic array 402 and haptic output devices associated with the mobile device may be utilized to output haptic effects to the user.

The haptic cane may output haptic effects via haptic array 402. These haptic effects may comprise simple vibrations to alert the user of an obstacle. Alternatively, these haptic effects may comprise more complex effects configured to vary in intensity to alert the user to the type of obstacle, e.g., a strong effect for a dangerous obstacle such as an open manhole cover or an approaching vehicle and a weak haptic effect for a less dangerous obstacle such as a piece of furniture or another person.

Further, in the embodiment shown in Figure 4 the haptic cane 400 may be configured to direct the user along a path. Thus, for example, the haptic cane 400 may be configured to output haptic effects to alert the user to move forward, backward, left, or right. Further, if the user encounters stairs the haptic cane 400 may be configured to provide feedback indicating that the user should go up the stairs or down the stairs. In some embodiments these haptic effects may comprise effects output at different locations on the haptic array 402, e.g., an effect at the front of the haptic array 402 to direct the user to move forward and an effect at the rear of haptic array 402 to direct the user to move backward. In some embodiments, the haptic cane 400 may comprise Satellite Positioning System functionality and thus be able to provide the user directions between multiple relatively distant points.

In still other embodiments, systems and methods for tactile guidance may be used as a finding tool. For example, a user may use systems and methods disclosed herein to locate objects in an area in which the user cannot see, e.g., in a dark area or under another object (e.g., under the user's bed). In such an embodiment, systems and methods for tactile guidance may act as a detector that allows the user to determine if an object is in a searched area, e.g., the user may be use embodiments disclosed herein to determine that the user's cat is under a bed. In such an embodiment, the user may hold his or her phone under the bed. The area sensor may detect objects in the area, and if one of those objects is a searched for object (in this case the cat), the processor of the mobile device may determine a haptic effect to alert the user that the object is found. Such an embodiment may be useful for locating lost or misplaced objects (e.g., keys, remote control, tools, headphones, etc.).

### Illustrative Methods for Tactile Guidance

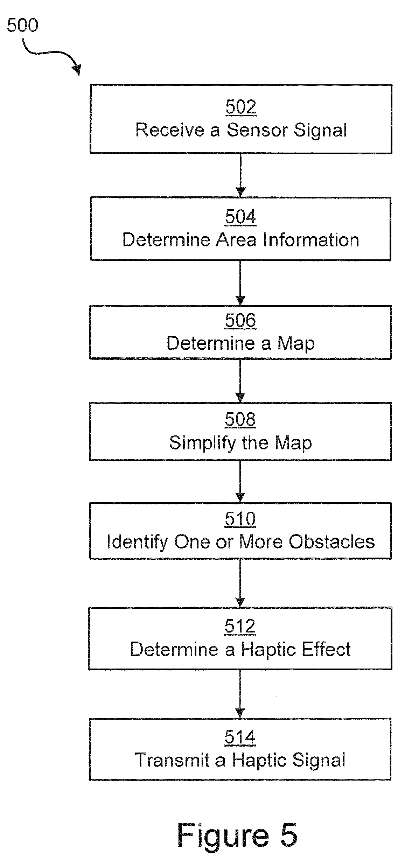

Figure 5 is a flow chart of steps for performing a method for tactile guidance according to one embodiment. In some embodiments, the steps in Figure 5 may be implemented in program code that is executed by a processor, for example, the processor in a general purpose computer, a mobile device, or a server. In some embodiments, these steps may be implemented by a group of processors. In some embodiments one or more steps shown in Figure 5 may be omitted or performed in a different order. Similarly, in some embodiments, additional steps not shown in Figure 5 may also be performed. The steps below are described with reference to components described above with regard to computing device 100 shown in Figure 1.

The method 500 begins at step 502, when processor 102 receives a sensor signal from an area sensor 115. As discussed above, area sensor(s) 115 comprise one or more devices configured to detect objects associated with the area around the user and transmit signals associated with these objects to the processor(s) 102. For example, area sensor(s) 115 may comprise one or more of an ultrasonic sensor, an infrared sensor, a laser sensor, or a camera. In one embodiment, area sensor(s) 115 may comprise an MB1320 XL-MaxSonar-AE2, however, in other embodiments many other types and models of area sensor(s) may be used as well or alternatively. In some embodiments, the objects detected by area sensor(s) 115 may comprise any object found in an area around the user, e.g., indoor objects (e.g., furniture, supporting posts, walls, doors, or other objects associated with an indoor area); outdoor objects (e.g., trees, rocks, holes, roots, stumps, curbs, cars, bicycles, or other objects associated with an outdoor area). Further, the objects may comprise moving objects (e.g., animals, cars, people, etc.) or non-moving objects (e.g., trees, walls, puddles, etc.). Thus, in one embodiment area sensor 115 may detect an object in the form of a wall and transmit a signal to processor 102 associated with the wall.

Next, processor 102 determines area information 504. In some embodiments the processor 102 determines area information based in part on the signal received from area sensor 115. The area information may comprise, for example, information about objects within a distance of the user. These objects may comprise the objects discussed, above, e.g., indoor or outdoor objects, some of which may comprise obstacles. In some embodiments the processor also determines a route along which the user is traveling or will travel. In some embodiments, this route is determined based in part on information received from a satellite positioning system.

In some embodiments the area sensor 115 may comprise a camera. In such an embodiment, the processor 102 may determine area information from the camera signal by smoothing the image received from the camera. The processor 102 may convert the signal from the RGB to HSV color space. The processor 102 may then create a threshold image. If a pixel in the HSV image is between threshold values the processor may copy the pixel to a threshold image. The processor 102 may then convert the threshold image to a 3D matrix. Then, if the number of non-zero elements in the 3D matrix are greater than a detection value the processor 102 may return that an object was found. Similarly, in some embodiments, the area sensor may be configured to detect key descriptors associated with the objects. For example, the area sensor 115 may comprise an infrared camera. In some embodiments, the infrared camera may detect thermal characteristics of one or more objects.

Next processor 102 determines a map 506. In some embodiments the map may comprise detail regarding the area information. This detail may comprise every object within an area of the user. In some embodiments, the processor 102 may store this map locally. Alternatively, in some embodiments, the processor may transmit the map to a remote data store for further processing and storage.

In some embodiments, the processor 102 may determine the map by receiving map data from a database, e.g., a locally stored database or a remote database accessed via a network connection. For example, in some embodiments the processor may determine the map by receiving data from a remote database accessible via the Internet. In some embodiments, such a database may comprise a "cloud" database, which may be continuously updated by one or more public or private groups. Further, such a database may comprise a database of map data used or accessed by a plurality of mobile applications and webpages. In some embodiments, the processor may determine the map by comparing information about the area around the user (e.g., information about the objects in the area) to information stored in a database. In some embodiments, the processor 102 may determine the user's current location by accessing the database.

Then processor 102 simplifies the map 508. In some embodiments, simplifying the map may comprise simplifying the area information to a collection of vectors to each object within an area of the user. This series of vectors may enable the processor 102 to access simplified data to determine how far a user may move in each direction before encountering an object.

Next, processor 102 identifies one or more obstacles 510. An obstacle may comprise any object discussed above that is in a user's route. Thus, for example, an obstacle may comprise one or more objects that might impede the user's forward progress if the user continued moving in the same direction.

In some embodiments, the processor 102 may continuously update information regarding objects and the map. For example, in some embodiments the user may be in motion. In such an embodiment, the processor may continue to monitor area sensor(s) 115 and maintain substantially up-to-date information regarding the user's location with respect to the objects. Similarly, in some embodiments the objects may comprise moving objects, such as, cars, buses, trains, animals, or other people, etc. In such an embodiment, the processor 102 may continue to monitor area sensor(s) 115 and maintain substantially up-to-date information regarding the user's location with respect to the objects.

Next the processor determines a haptic effect 512. In some embodiments, the processor 102 may determine a haptic effect based on user selection. For example, the user may select an available haptic effect to be output when the user encounters an object. For example, a data store of computing device 101 may comprise data associated with multiple haptic effects, which the user may select. Alternatively, in some embodiments, the processor 102 may automatically select the haptic effect. For example, in some embodiments, the processor 102 may select a haptic effect associated with objects detected by area sensors 115. For example, the processor may determine a more intense haptic effect for more dangerous objects, e.g., fast moving objects (e.g., cars, buses, trains) and less intense haptic effects for more distant or less dangerous objects, e.g., furniture or people.

In some embodiments the processor 102 may be configured to determine whether to output an alert based on one or more key descriptors associated with a detected object. In such an embodiment, the key descriptors may comprise, e.g., the object's size, direction of movement, speed of movement, distance from the user, relative danger, or some other key descriptor associated with the object. For example, if the detected object is relatively distant (e.g., more than a threshold distance), the illustrative device may output no alert. Similarly, the illustrative device may determine additional information, about the object, e.g., the object's size, its relative danger, its speed of movement, its direction of movement, and based on this information determine whether to output an alert to the user.

Further, in some embodiments, based on one or more key descriptors associated with the object the processor 102 may determine not to output any form of alert (e.g., no audible alert, visual alert, or haptic effect). For example, based on the sensor signal the processor 102 may determine key descriptors associated with the object such as the object's distance, the speed the object is moving, the direction of movement, the size of the object, or some other characteristic associated with the object, and based on this information determine not to output any form of alert. For example, the processor may determine that a bus moving away from the user requires no alert, whereas a bus moving toward the user requires an alert. Similarly, in some embodiments, the processor 102 may determine not to output an alert based on the size of an object, e.g., the processor 102 may determine no alert for a very small object, like a paperclip, in the user's path. Further, the processor 102 may determine not to output an alert based on the type of object. For example, the processor 102 may determine not to output an alert for a small docile dog, but to output an alert for a large aggressive dog. In a further embodiment, the processor 102 may determine not to alert the user because an object is detected as relatively distant from the user, e.g., more than a threshold distance. In such an embodiment, if an object is located more than the threshold distance the processor 102 may determine not to output an alert.

Then the processor 102 transmits a haptic signal to haptic output device 118, which outputs the haptic effect 514. As discussed above, the haptic effect may comprise a texture (e.g., sandy, bumpy, or smooth), a vibration, a change in a perceived coefficient of friction, a change in temperature, a stroking sensation, an electro-tactile effect, or a deformation (e.g., a deformation of a surface associated with the computing device 101).

There are numerous advantages of tactile guidance. For example, tactile guidance may allow users to more fully engage with mobile applications without fear of dangerous or embarrassing collisions. This may increase user safety and also increase user satisfaction. Further, embodiments of the present disclosure may provide additional mobility to people with visual impairments. Further, embodiments of the present disclosure may provide tools for use by people that often must operate in visually restrictive areas, e.g., soldiers, police, firemen, etc. Any one of these features provides additional uses of mobile devices and mobile applications to a broad set of users.

### General Considerations

The methods, systems, and devices discussed above are examples. Various configurations may omit, substitute, or add various procedures or components as appropriate. For instance, in alternative configurations, the methods may be performed in an order different from that described, and/or various stages may be added, omitted, and/or combined. Also, features described with respect to certain configurations may be combined in various other configurations. Different aspects and elements of the configurations may be combined in a similar manner. Also, technology evolves and, thus, many of the elements are examples and do not limit the scope of the disclosure or claims.

Specific details are given in the description to provide a thorough understanding of example configurations (including implementations). However, configurations may be practiced without these specific details. For example, well-known circuits, processes, algorithms, structures, and techniques have been shown without unnecessary detail in order to avoid obscuring the configurations. This description provides example configurations only, and does not limit the scope, applicability, or configurations of the claims. Rather, the preceding description of the configurations will provide those skilled in the art with an enabling description for implementing described techniques. Various changes may be made in the function and arrangement of elements without departing from the spirit or scope of the disclosure.

Also, configurations may be described as a process that is depicted as a flow diagram or block diagram. Although each may describe the operations as a sequential process, many of the operations can be performed in parallel or concurrently. In addition, the order of the operations may be rearranged. A process may have additional steps not included in the figure. Furthermore, examples of the methods may be implemented by hardware, software, firmware, middleware, microcode, hardware description languages, or any combination thereof. When implemented in software, firmware, middleware, or microcode, the program code or code segments to perform the necessary tasks may be stored in a non-transitory computer-readable medium such as a storage medium. Processors may perform the described tasks.

Having described several example configurations, various modifications, alternative constructions, and equivalents may be used without departing from the spirit of the disclosure. For example, the above elements may be components of a larger system, wherein other rules may take precedence over or otherwise modify the application of the invention. Also, a number of steps may be undertaken before, during, or after the above elements are considered. Accordingly, the above description does not bind the scope of the claims.

The use of "adapted to" or "configured to" herein is meant as open and inclusive language that does not foreclose devices adapted to or configured to perform additional tasks or steps. Additionally, the use of "based on" is meant to be open and inclusive, in that a process, step, calculation, or other action "based on" one or more recited conditions or values may, in practice, be based on additional conditions or values beyond those recited. Headings, lists, and numbering included herein are for ease of explanation only and are not meant to be limiting.

Embodiments in accordance with aspects of the present subject matter can be implemented in digital electronic circuitry, in computer hardware, firmware, software, or in combinations of the preceding. In one embodiment, a computer may comprise a processor or processors. The processor comprises or has access to a computer-readable medium, such as a random access memory (RAM) coupled to the processor. The processor executes computer-executable program instructions stored in memory, such as executing one or more computer programs including a sensor sampling routine, selection routines, and other routines to perform the methods described above.

Such processors may comprise a microprocessor, a digital signal processor (DSP), an application-specific integrated circuit (ASIC), field programmable gate arrays (FPGAs), and state machines. Such processors may further comprise programmable electronic devices such as PLCs, programmable interrupt controllers (PICs), programmable logic devices (PLDs), programmable read-only memories (PROMs), electronically programmable read-only memories (EPROMs or EEPROMs), or other similar devices.

Such processors may comprise, or may be in communication with, media, for example tangible computer-readable media, that may store instructions that, when executed by the processor, can cause the processor to perform the steps described herein as carried out, or assisted, by a processor. Embodiments of computer-readable media may comprise, but are not limited to, all electronic, optical, magnetic, or other storage devices capable of providing a processor, such as the processor in a web server, with computer-readable instructions. Other examples of media comprise, but are not limited to, a floppy disk, CD-ROM, magnetic disk, memory chip, ROM, RAM, ASIC, configured processor, all optical media, all magnetic tape or other magnetic media, or any other medium from which a computer processor can read. Also, various other devices may include computer-readable media, such as a router, private or public network, or other transmission device. The processor, and the processing, described may be in one or more structures, and may be dispersed through one or more structures. The processor may comprise code for carrying out one or more of the methods (or parts of methods) described herein.

While the present subject matter has been described in detail with respect to specific embodiments thereof, it will be appreciated that those skilled in the art, upon attaining an understanding of the foregoing may readily produce alterations to, variations of, and equivalents to such embodiments. Accordingly, it should be understood that the present disclosure has been presented for purposes of example rather than limitation, and does not preclude inclusion of such modifications, variations and/or additions to the present subject matter as would be readily apparent to one of ordinary skill in the art. Embodiments of the present invention have been described with particular reference to the examples illustrated. However, it will be appreciated that variations and modifications may be made to the examples described within the scope of the present invention.

## Claims

1. A method for tactile guidance comprising:
receiving a sensor signal from a sensor (115) configured to determine one or more objects associated with an area;
determining area information based in part on the sensor signal;
determining a haptic effect based in part on the area information; and
transmitting a haptic signal associated with the haptic effect to a haptic output device (118) configured to output the haptic effect.

2. The method of claim 1, further comprising:
determining a map (506) based in part on the sensor signal; and
transmitting data associated with the map to a remote database.

3. The method of claim 2, wherein determining the map (506) comprises receiving data about the map from a database.

4. The method of claim 2, further comprising:
simplifying the map to comprise linear distances within the area;
identifying one or more obstacles in the area.

5. The method of any one or more of claims 1-4, wherein the haptic effect is associated with the one or more obstacles.

6. The method of any one or more of claims 1 - 5, wherein the sensor (115) comprises one or more of an ultrasonic sensor, an infrared sensor, a laser sensor, or a camera.

7. The method of any one or more of claims 1 - 6, wherein the haptic output device (118) comprises one or more of a piezoelectric actuator, an electric motor, an electro-magnetic actuator, a voice coil, a shape memory alloy, an electro-active polymer, a solenoid, an eccentric rotating mass motor (ERM), or a linear resonant actuator (LRA).

8. The method of any one or more of claims 1 - 7, wherein the sensor (115) and haptic output device (118) are both associated with one of a wearable or a graspable device.

9. The method of claim 8, wherein the graspable device comprises one of a mobile device or a cane.

10. The method of claim 8, wherein the wearable device comprises one of a helmet, gloves, glasses, or augmented reality glasses.

11. A non-transitory computer readable medium comprising program code, which when executed by the processor (102) is configured to cause the processor to give effect to the method as claimed in any one of claims 1-10.

12. A system (100) for tactile guidance comprising:
a sensor (115) configured to determine area information and transmit a sensor signal associated with the area information;
a processor (102) in communication with the sensor and configured to:
determine area information based in part on the sensor signal;
determine a haptic effect based in part on the area information; and
transmit a haptic signal associated with the haptic effect;
a haptic output device (118) in communication with the processor, the haptic output device configured to receive the haptic signal and output the haptic effect.

13. The system (100) of claim 12, wherein all of the components of the system are associated with a cane.
